# EUROPEAN PATENT SPECIFICATION

(11) **EP 2 296 486 B1**
(45) Date of publication and mention of the grant of the patent: **29.04.2015**
(21) Application number: 08748809.4
(22) Date of filing: 26.05.2008
(51) Int. Cl.: A23G 4/20, A23G 4/18, A61K 9/00

(54) **FILM-COATED COMPRESSED CHEWING GUM**
DRAGIERTER KOMPRIMIERTER KAUGUMMI
CHEWING-GUM COMPRIMÉ ET ENROBÉ

(43) Date of publication of application: 23.03.2011
(73) Proprietor: Fertin Pharma A/S, 7100 Vejle (DK)
(72) Inventor: NIELSEN, Bruno, Provstgaard, DK-7120 Vejle Øst (DK); DALHOFF, Jan, DK-8722 Hedensted (DK); LORENZEN, Gitte, DK-7120 Vejle Øst (DK)
(74) Representative: Patentgruppen
(86) International application number: PCT/DK2008/000198
(87) International publication number: WO 2009/143841

(56) References cited:
- WO-A-2004/004479
- WO-A-2004/004480
- WO-A-2004/068965
- WO-A-2005/063038
- WO-A-2006/002622
- WO-A-2006/124366
- WO-A-2007/090426
- WO-A-2007/109718
- US-A- 5 098 715
- US-A1- 2002 164 398

## Description

### FIELD OF THE INVENTION

The invention relates to chewing gum, more particularly to the field of compressed chewing gum tablets.

### TECHNICAL BACKGROUND AND PRIOR ART

Compressed chewing gum tablets are characterized in that a considerable amount of water soluble chewing gum ingredients is released immediately upon the first few bites into the chewing gum tablet. This initial burst of sweetness may not be a problem by itself, but the very short period of a very intense taste sensation may often lead to disappointment as the chewing continues, because there may be a mismatch between the user's expectations of a prolonged intense taste and the actual more moderate taste release from the chewing gum after the initial chewing phase of about 30 seconds.

It has been a problem to moderate, alter or counteract the initial burst of taste from a compressed chewing gum tablet.
US 5,098,715 to McCabe et al. discloses a pharmaceutical tablet comprising an unpleasant tasting solid core and a flavored pharmaceutically acceptable thin film coating.
WO 2006/124366 discloses a chewing gum comprising a core; at least one active pharmaceutical ingredient (API) within the core; at least one inner polymer film coating applied onto the core; and at least one outer hard coating applied onto the outermost inner polymer film coating.
US 6,444,241 to Tyrpin et al. discloses a method for producing a chewing gum with a controlled release of caffeine. Caffeine is incorporated into a sugar or a polyol coating.
US 2003/0198713 A1 discloses a confectionary tablet with multiple coatings leading to a desired flavor release.

It is an objective of the present invention to provide a compressed chewing gum tablet providing a moderated initial burst of taste from the chewing gum upon chewing.

### SUMMARY OF THE INVENTION

The invention relates to a compressed chewing gum tablet according to claim 1 comprising at least one compressed chewing gum module, the at least one compressed chewing gum module including a compressed particulate chewing gum composition, which compressed particulate chewing gum composition comprising compressed chewing gum particles containing gum base, wherein the content of gum base is at least 5 % by weight of the tablet, and wherein the chewing gum tablet is provided with a film coating, and wherein the film coating comprises liquid flavoring.

In prior art effort has been put into manufacturing compressed chewing gum tablets exercising a satisfying initial flavor burst. The result has been chewing gum tablets that, in the eyes of a typical chewing gum consumer, are satisfying only for a very short chewing period compared to conventional chewing gum.

According to the present invention, it is possible to alter the initial burst of flavor by coating the compressed chewing gum tablet with a film coating comprising liquid flavor.

Surprisingly it has been found that adding liquid flavor to a compressed chewing gum tablet in a film coating surrounding the tablet can moderate the taste sensation in a way as to make the chewing gum satisfactorily usable for a prolonged period of time.
The role of the film coating comprising liquid flavor is at least twofold:
a) The film coating helps to keep the chewing gum granules together in the initial chewing period, thereby somewhat lessening the immediate release of sweeteners and flavors from the compressed chewing gum tablet.
b) The liquid flavor in the film coating matches the intense sweetness provided by the compressed chewing gum tablet thereby making the taste sensation less extreme and the moderated taste after the initial chewing period more acceptable.

Advantageously, concentrated liquid flavors are used in the film coating to maximize the impact of the taste release from the film coating.

According to the invention, the amount of liquid flavor in the chewing gum tablet, including the film coating, is above 0.05 % by weight of the chewing gum tablet.

It may be difficult to use liquid flavoring in a chewing gum tablet, because the addition of liquids to the chewing gum particles may compromise the compression process and ultimately lead to disintegration of the tablet.
According to provisions of the invention, liquid flavoring can be added to a chewing gum tablet by coating the tablet with a film coating comprising liquid flavoring. Liquid flavoring compositions which may not have been available in the prior art of chewing gum tablet manufacture can, according to provisions of the invention, become an integral part of the tablet without this leading to disintegration. Surprisingly it has been found by the inventors that a film coating process, in which the film coating comprises liquid flavoring, is applicable to chewing gum tablets without compromising the integrity of the compressed chewing gum tablet. On the contrary, the film coating layer comprising liquid flavoring may act as a protective barrier preserving the shape and the character of the chewing gum tablet.

In an embodiment of the invention, the amount of liquid flavor in the chewing gum tablet, excluding the film coating, is below 2 %, such as below 1%, by weight of the chewing gum tablet.

In an embodiment of the invention, any liquid flavor in the chewing gum tablet, excluding the film coating, is substantially located in the compressed chewing gum particles containing gum base.

Any liquid flavor in the chewing gum tablet, excluding the film coating, may be substantially located in the compressed chewing gum particles containing gum base, and any additional flavor in the chewing gum tablet, excluding the film coating, may be substantially in the form of particulate flavor.

In an embodiment of the invention, said compressed chewing gum tablet comprises at least one active pharmaceutical ingredient.

In an advantageous embodiment of the invention the compressed chewing gum tablet comprises an active pharmaceutical ingredient (API). The use of liquid flavoring in a film coating surrounding the compressed material may effectively mask the sometimes unpleasant taste of the API in the initial chewing phase.
Furthermore, it may be advantageous to keep liquid flavoring and API separate in the manufacturing process and during storage of the final compressed chewing gum tablet to avoid chemical reactions between flavor and API which may result in API modification and loss of API activity.

In an embodiment of the invention, said film coating is applied to said compressed chewing gum tablet prior to any optional coating.

Preferably, the film coating is applied directly on the compressed chewing gum tablet to ensure good protection of the tablet against moisture from the environment or from further processing steps.

The amount of liquid flavor in the chewing gum tablet, excluding the film coating, may be below 2 %, such as below 1 %, by weight of the chewing gum tablet and the amount of liquid flavor in the film coating may be above 0.5% by weight of the tablet, including the film coating.

In an embodiment of the invention, the liquid flavor in the film coating constitutes at least 10% (w/w), preferably at least 20% (w/w) of the total flavor content of the film coated chewing gum tablet.

Said film coating may comprise natural polymers.

Said film coating may comprise synthetic polymers.

Said film coating may have a dry thickness from about 15 to about 110 micrometer.

The desired thickness of the film coating layer may vary. Depending on film-forming polymers, flavor type and other factors, thinner or thicker film coating layers may be advantageous.

In an embodiment of the invention, said liquid flavoring is selected from the group consisting of coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, mint, fruit essence such as from apple, pear, peach, strawberry, apricot, raspberry, cherry, pineapple, and plum essence, essential oils including peppermint, spearmint, menthol, eucalyptus, clove oil, bay oil, anise, thyme, cedar leaf oil, nutmeg, oils of the fruits mentioned above and combinations thereof.

The use of liquid flavorings in the film coating may give rise to several advantages when combined with compressed chewing gum. Powdery flavors are often more expensive and less concentrated than liquid flavors and certain limits of their content in a compressible formulation have to be acknowledged to ensure compressibility. Furthermore, since most flavors are hydrophobic liquids, the direct use of these in a film coating formulation may give access to materials which may not be applicable directly in a compressible formulation.

According to the invention, said film coating comprises liquid flavoring in an amount of more than 1%, preferably more than 5% by weight of the dry film coating.

Said film coating may comprise liquid flavoring in an amount of more than 10%, preferably more than 20% by weight of the dry coating.

Said natural polymers may be selected from the group consisting of shellac, cellulose, zein, starch, gelatin, vegetable gum, saccharide compounds, dextrins, polydextrose or combinations thereof.

Said synthetic polymers may be selected from the group consisting of cellulose ethers, acrylic polymers, cellulose acetate phthalate (CAP), Polyvinyl acetate phthalate (PVAP), Cellulose acetate trimellitate (CAT), Hydroxyethyl cellulose (HEC), Hydroxypropyl cellulose (HPC), Hydroxypropyl methylcellulose (HPMC), Hydroxy methylcellulose phthalate (HPMCP), Ethylcellulose (EC), Methacrylate ester copolymers, Methacrylic acid copolymers or combinations thereof.

Said natural and/or synthetic polymers may be water-soluble.

In an embodiment of the invention the polymers used as film forming agents are either water soluble or made water soluble by dispersion techniques. The use of water as solvent in the film coating process reduces or eliminates the use of organic solvents which may impose environmental concerns.

Said natural and/or synthetic polymers may be water-insoluble.

In an embodiment of the invention, said film coating confers a modified release performance with respect to active components of the chewing gum formulation.

The film coating may modify the release of active ingredients from the chewing gum core. Especially in the initial chewing phase, the film coat may moderate instant access to the sweeteners, flavoring agents etc. comprised in the chewing gum tablet thereby moderating the initial burst of taste associated with chewing gum tablets.

The liquid flavoring comprised in the film coating may at the same time supplement the initial burst of taste from the chewing gum tablet core thereby providing a more balanced flavor sensation for the consumer of the chewing gum.

Said film coating may comprise plasticizer.

It may be advantageous to soften the film forming polymers by using a plasticizer in the film coating.

In an embodiment of the invention, said film coating comprises high intensity sweetener.

The optional coating may be a hard coating.

According to provisions of the invention the film coating layer may additionally act as a primer for further hard coating of the compressed chewing gum tablet.

In an embodiment of the invention, said at least one active pharmaceutical ingredient is nicotine.

In an embodiment of the invention, said at least one active pharmaceutical ingredient is selected from the group consisting of cetirizine, levo cetirizine, metformin, metformin HCL, phenylephrine, GLP-1, exenatide, MC-4 receptor antagonist, PPY(3-36), deca-peptide, KSL-W (acetate), fluor, and chlorhexidine.

Said hard coating may comprise at least one flavoring agent.

An advantageous initial flavor release profile may be achieved by combining the liquid flavoring agents in the film coating with flavoring agents in a further hard coating.

In an embodiment of the invention, said film coating comprises at least one active pharmaceutical ingredient.

Combining liquid flavor and API in the film coating may be advantageous in cases where comparatively fast initial release of API is required and at the same time, masking the taste of the API with concentrated flavor is important.

Said compressed chewing gum tablet may comprise particles free of gum base.

Chewing gum ingredients may be mixed with gum base granules in a compressible composition.
Favorable release profiles for sweeteners, flavors etc. may be achieved by combining gum base ingredients in the gum base particles with chewing gum ingredients outside the gum base-containing particles.

Said chewing gum particles containing gum base may comprise further chewing gum ingredients.

Said chewing gum particles free of gum base may comprise chewing gum ingredients.

Said chewing gum particles free of gum base may comprise flavor.

Said chewing gum particles free of gum base may constitute at least 40% by weight of the compressed chewing gum tablet.

Said chewing gum particles free of gum base may constitute at least 60% by weight of the compressed chewing gum tablet.

Said compressed chewing gum tablet may consist of one module.

Said compressed chewing gum tablet may comprise at least one gum base-free module.

Said compressed chewing gum tablet may comprise at least two modules.

Said compressed chewing gum tablet may comprise at least one gum base-free module and at least one gum base-containing module.

The gum base-free module may constitute more than 50% by weight of the chewing gum tablet.

The ratio measured in % by weight of the whole tablet between the at least one gum base-containing module and the at least one gum base-free module may be at least 65:35.

Said compressed chewing gum tablet may comprise high intensity sweeteners in an amount of less than 1, preferably less than 0.5% by weight of the said compressed chewing gum tablet.

Said compressed chewing gum tablet may comprise high intensity sweeteners in an amount of more than 0.05% by weight of the said compressed chewing gum tablet.

Said compressed chewing gum tablet may comprise bulk sweetener in an amount of at least 10%, preferably at least 15% and most preferably at least 20% by weight of the chewing gum tablet.

The release of sweetener from a compressed chewing gum may be very intense in the initial chewing phase. In an advantageous embodiment of the invention, the liquid flavor comprised in the film coating surrounding the compressed chewing gum tablet may be used to complement the burst of sweetness from the compressed chewing gum thus concerting the flavor- and sweetness sensation experienced by the user of the chewing gum.

In an embodiment of the invention, said compressed chewing gum tablet comprises biodegradable polymers.

In an embodiment of the invention at least a part of the chewing gum polymers are biodegradable. This may lead to lessening of the problems associated with dropping the chewing gum in the environment after use.

Said compressed chewing gum tablet may have a volume of at least 0.15 cm³.

Said compressed chewing gum tablet may be compressed by a force of more than 13 kN.

Moreover the invention relates to a method for production of a chewing gum according to any of the claims 1-45, wherein said method comprises at least one compression step and at least one film coating step.

### DETAILED DESCRIPTION

With the present invention, as described in the following, a compressed chewing gum tablet having a film coating comprising liquid flavor is provided.
It is obtained that the taste of the chewing gum tablet in the initial chewing phase is modified in such a way as to achieve an improved match between the release of sweetener and flavor from the tablet.

In accordance with the general principles in manufacturing a chewing gum tablet within the scope of the invention, variations of different suitable ingredients are listed and explained below.

Chewing gum of the present invention typically comprises a water-soluble portion, a water-insoluble chewable gum base portion and flavoring agents. The water-soluble portion dissipates with a portion of the flavoring agent over a period of time during chewing. The gum base portion is retained in the mouth throughout the chew. The term chewing gum refers to both a chewing and bubble type gum in its general sense.

The gum base is the masticatory substance of the chewing gum, which imparts the chew characteristics to the final product.

The insoluble portion of the gum typically may contain any combination of elastomers, vinyl polymers, elastomer plasticizers, waxes, softeners, fillers and other optional ingredients such as colorants and antioxidants.

The composition of gum base formulations can vary substantially depending on the particular product to be prepared and on the desired masticatory and other sensory characteristics of the final product. However, typical ranges (% by weight) of the above gum base components are: 5 to 80% by weight elastomeric compounds, 5 to 80% by weight elastomer plasticizers, 0 to 40% by weight of waxes, 5 to 35% by weight softener, 0 to 50% by weight filler, and 0 to 5% by weight of miscellaneous ingredients such as antioxidants, colorants, etc. The gum base may comprise about 5 to about 95 percent, by weight, of the chewing gum, more commonly the gum base comprises 10 to about 60 percent, by weight, of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in base. This may be important when one wants to provide more elastomeric chain exposure to the alkane chains of the waxes.

The elastomer compounds may be of natural origin but are preferably of synthetic origin, preferably synthetic polyesters.

It is noted that gum base or gum granules may also include components typically referred to as chewing gum ingredients.
The chewing gum may, according to examples of the invention, comprise conventionally non-biodegradable polymers, such as natural resins, synthetic resins and/or synthetic or natural elastomers.

According to an example, at least a part of the polymers of the chewing gum are biodegradable.

In an example, the chewing gum may comprise combinations of biodegradable polymers and polymers generally regarded as non-biodegradable, such as natural resins, synthetic resins and/or synthetic/natural elastomers.

In an example, said natural resin comprises terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

Examples of generally non-biodegradable synthetic resins include polyvinyl acetate, vinyl acetate-vinyl laurate copolymers and mixtures thereof. Examples of non-biodegradable synthetic elastomers include, but are not limited to, synthetic elastomers listed in Food and Drug Administration, CFR, Title 21, Section 172,615, Masticatory Substances, Synthetic specification, such as polyisobutylene. e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

The elastomers (rubbers) employed in the gum base may vary depending upon various factors such as the type of gum base desired, the texture of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate, and the like, and mixtures thereof.

It is common in the industry to combine in a gum base a synthetic elastomer having a high molecular weight with a synthetic elastomer having a low molecular weight. Examples of such combinations are polyisobutylene with styrene-butadiene, polyisobutylene with polyisoprene, polyisobutylene with isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Examples of natural resins are: Natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

The compressed chewing gum tablet according to the invention is provided with a film coating comprising at least one component selected from the group consisting of an edible film-forming agent and a wax.
In the present context, suitable film-coating polymers include natural polymers and synthetic polymers. Natural polymers include Shellac, Zein, Cellulose, Starch, High amylose starch, Gelatin, Vegetable gum, Saccharide compounds, Dextrins, Polydextrose, Pullulan, Tragacanth gum, Guar gum, Acacia gum, Arabic gum, Amylose, High amylase starch, Pectin, Chitin, Gluten, Soy protein isolate, Whey protein isolate, Casein, Hydrogenated vegetable oils, Hydrogenated castor oils, Gum rosins and Wood rosins.
Synthetic polymers include Cellulose derivatives such as Cellulose ethers including Methyl cellulose (MC), Hydroxyethyl cellulose (HEC), Hydroxypropyl cellulose (HPC), Hydroxyethyl methylcellulose, Hydroxypropyl methylcellulose phthalate (HPMCP), Cellulose acetate and hydroxypropyl methylcellulose (HPMC). Other useful synthetic film-coating agents are acrylic polymers and copolymers, e.g. Polymethacrylates, Methylacrylate ester copolymers, Methacrylic acid copolymers or mixtures of cellulose derivatives and acrylic polymers.
Further useful film coating polymers include Sodium alginate, Ammonium alginate, Hydroxypropylated high amylase starch, Chitosan, Copovidone, Dimethylphthalate, Ethyl lactate, Hypromellose acetate succinate, Maltodextrin, Polyethylene glycol (PEG), Polyethylene oxide, Poly(methylvinyl ether/maleic anhydride), Carboxymethylcellulose sodium, Glyceryl behenate, Glyceryl Palmitostearate, Poloxamer, Polyvinyl alcohol, Povidone (Polyvinylpyrrolidone, PVP), Polyvinyl chloride and Polyurethane. A particular group of film-coating polymers also referred to as functional polymers are polymers that, in addition to its film-forming characteristics, confer a modified release performance with respect to active components of the chewing gum formulation. Such release modifying polymers include methylacrylate ester copolymers, ethylcellulose (EC) and enteric polymers designed to resist the acidic stomach environment, yet dissolve readily in the duodenum. The latter group of polymers includes: cellulose acetate phthalate (CAP), polyvinyl acetate phthalate (PVAP), shellac, metacrylic acid copolymers, cellulose acetate trimellitate (CAT) and HPMC. It will be appreciated that the film coating according to the present invention may comprise any combination of the above film-coating polymers.

The choice of film-forming polymer(s) and plasticizing agent(s) for the film coating of the compressed chewing gum tablet is made with due consideration for achieving the best possible barrier properties of the coating in respect of dissolution and diffusion across the film of moisture and gasses.

Suitable plasticizing agents for the film coating include Dibuthylphthalate, Dibuthylsebacate, Diethylphthalate, Triethyl citrate, Tributhyl citrate, Acetyl triethyl citrate, Acetyl tributhyl citrate, Triacetin (glycerol triacetate), Acetyltributhyl citrate, Acetyltriethyl citrate, Glycerol, Propylene glycol, Polyethylene glycol (200 - 6000 Grades), Dibuthyl sebacete, Castor oil, Acetylated monoglycerides and Fractionated coconut oil.

The film coating of the chewing gum tablet may also comprise one or more colorants or opacifiers. In addition to providing a desired color hue, such agents may contribute to protecting the compressed gum base against pre-chewing reactions, in particular by forming a barrier against moisture and gasses. Suitable colorants/pacifiers include organic dyes and their lakes, inorganic coloring agents, e.g. titanium oxide and natural colors such as e.g. β-carotene.

Additionally, film coatings may comprise one or several auxiliary substances such as waxes, anti-foaming agents, surfactants, preservatives, anti-tack agents and/or saccharide compounds such as polydextrose, dextrins including maltodextrin, lactose, modified starch, a protein such as gelatine or zein, a vegetable gum and any combination thereof.

The film-forming agent may further be selected from the group comprising cellulose derivative, a modified starch, a dextrin, gelatine, shellac, gum arabic, zein, a vegetable gum, a synthetic polymer and any combination thereof.
According to the invention, the film coating comprises liquid flavoring.

Examples of flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

According to examples of the invention the film coating comprises at least one additive component selected from the group comprising of a binding agent, a moisture-absorbing component, a film-forming agent, a dispersing agent, an antisticking component, a bulking agent, a powdery flavoring agent, a coloring agent, a pharmaceutically or cosmetically active component, a lipid component, a wax component, a sugar, an acid and an agent capable of accelerating the after-chewing degradation of the degradable polymer.

According to examples of the invention the film coated compressed chewing gum tablet may be coated with further coating layers, e.g. a hard coating.
The applicable hard coating may be selected from the group comprising sugar coating, a sugarless coating and a combination thereof. The hard coating may e.g. comprise 50 to 100% by weight of a polyol selected from the group consisting of sorbitol, maltitol, mannitol, xylitol, erythritol, lactitol and isomalt and variations thereof.

In the manufacturing of conventional chewing gum, the ingredients, generally, may be mixed by first melting the gum base and adding it to the running mixer. Colors, active agents and/or emulsifiers may also be added at this time. A softener such as glycerin may also be added at this time, along with syrup and a portion of the bulking agent/sweetener. Further portions of the bulking agent/sweetener may then be added to the mixer. A flavoring agent is typically added with the final portion of the bulking agent/sweetener. A high-intensity sweetener is preferably added after the final portion of bulking agent and flavor has been added.

The entire mixing procedure typically takes from five to fifteen minutes, but longer mixing times may sometimes be required. Those skilled in the art will recognize that many variations of the above-described procedure may be followed, including the one-step method described in US patent application 2004/0115305 hereby incorporated by reference. Chewing gum is formed by extrusion, compression, and/or rolling and may be centre filled with liquids and/or solids in any form.

When manufacturing a compressed chewing gum tablet another method is applied, which is basically very different from the above described, but may broadly be described as an initial conventional mixing of the gum base, as above described, followed by a granulation of the obtained gum base mix. The obtained chewing gum granules may then be mixed with further chewing gum ingredients, such as sweeteners and flavor. This final granule mix may then be compressed into a chewing gum tablet under high pressure and typically with cooling applied. For each compression a layer is made and in this way it is possible to make multi-layered chewing gum, such as two, three or four layers, wherein each layer may include an individual composition, i.e. different active ingredients may be used for medical purposes or different colors may be used for visual purposes, etc.

In further examples of the present invention, the film coated compressed chewing gum tablet may also be provided with a further outer coating, which may be a hard coating, a soft coating, a further film coating, or a coating of any type that is known in the art, or a combination of such coatings. The total amount of coating may typically constitute 0.1 to 75% by weight of a coated chewing gum piece.

One preferred further outer coating type is a hard coating, which term is including sugar coatings and sugar-free (or sugarless) coatings and combinations thereof. The object of hard coating is to obtain a sweet, crunchy layer, which is appreciated by the consumer. In a typical process of providing the film coated compressed chewing gum tablets with a sugar coating the gum tablets are successively treated in suitable coating equipment with aqueous solutions of crystallizable sugar such as sucrose or dextrose, which, depending on the stage of coating reached, may contain other functional ingredients, e.g. fillers, colors, etc.

Preferably, the coating agent may be applied in a hard coating process is a sugarless coating agent, e.g. a polyol including as examples sorbitol, maltitol, mannitol, xylitol, erythritol, lactitol and isomalt or e.g. a mono- di-saccharide including as example trehalose.

Or alternatively a sugar-free soft coating e.g. comprising alternately applying to the film coated compressed chewing gum tablets a syrup of a polyol or a mono- di-saccharide, including as examples sorbitol, maltitol, mannitol, xylitol, erythritol, lactitol, isomalt and trehalose.

In an example, the further outer coating comprises at least one additive component selected from the group comprising a binding agent, a moisture-absorbing component, a film-forming agent, a dispersing agent, an anti-sticking component, a bulking agent, a flavoring agent, a coloring agent, a pharmaceutically or cosmetically active component, a lipid component, a wax component, a sugar, and an acid.

A compressed chewing gum tablet according to examples of the invention may have any form, shape or dimension that permits the chewing gum tablet to be coated using any conventional coating process.

In examples of the invention, conventional elastomers or resins may be supplemented or substituted by biodegradable polymers.

Biodegradable polymers that may be used in the chewing gum of the present invention may be homopolymers, copolymers or terpolymers, including graft- and block-polymers.

Useful biodegradable polymers, which may be applied as gum base polymers in the chewing gum of the present invention, may generally be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed.

The usually preferred polyfunctional alcohols contain 2 to 100 carbon atoms as for instance polyglycols and polyglycerols.

Gum base polymers may both be resinous and elastomeric polymers.

Suitable biodegradable gum base polymers include polyesters, polycarbonates, polyesteramides, polyesterurethanes, polyamides, prolamine, and combinations thereof.

The chewing gum may include any component known in the chewing gum art. For example, the chewing gum may include elastomers, bulking agents, waxes, elastomer solvents, emulsifiers, plasticizers, fillers, and mixtures thereof.

The chewing gum according to examples of the invention may comprise coloring agents. According to an example, the chewing gum may comprise color agents and whiteners such as FD&C-type dyes and lakes, fruit and vegetable extracts, titanium dioxide and combinations thereof.

Further useful chewing gum base components include antioxidants, e.g. butylated hydroxytoluene (BHT), butyl hydroxyanisol (BHA), propylgallate and tocopherols, and preservatives.

A gum base formulation may, in accordance with the present invention, comprise one or more softening agents e.g. sucrose esters including those disclosed in WO 00/25598, which is incorporated herein by reference, tallow, hydrogenated tallow, hydrogenated and partially hydrogenated vegetable oils, cocoa butter, degreased cocoa powder, glycerol monostearate, glyceryl triacetate, lecithin, mono-, di- and triglycerides, acetylated monoglycerides, lanolin, sodium stearate, potassium stearate, glyceryl lecithin, propylene glycol monostearate, glycerine, fatty acids (e.g. stearic, palmitic, oleic and linoleic acids) and combinations thereof. As used herein the term "softener" designates an ingredient, which softens the gum base or chewing gum formulation and encompasses waxes, fats, oils, emulsifiers, surfactants and solubilizers.

To soften the gum base further and to provide it with water-binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Useful emulsifiers can include, but are not limited to, glyceryl monostearate, propylene glycol monostearate, mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like and mixtures thereof are examples of conventionally used emulsifiers which can be added to the chewing gum base. In case of the presence of a biologically or pharmaceutically active ingredient as defined below, the formulation may comprise certain specific emulsifiers and/or solubilizers in order to disperse and release the active ingredient.

Waxes and fats are conventionally used for the adjustment of the texture and for softening of the chewing gum base when preparing chewing gum bases. In connection with the present invention, any conventionally used and suitable type of natural and synthetic wax and fat may be used, such as for instance rice bran wax, polyethylene wax, petroleum wax (refined paraffin and microcrystalline wax), sorbitan monostearate, tallow, propylene glycol, paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

A chewing gum base formulation may, if desired, include one or more fillers/texturisers including as examples, magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

In addition to a water insoluble gum base portion, a typical chewing gum includes a water soluble bulk portion and one or more flavoring agents. The water-soluble portion may include bulk sweeteners, high-intensity sweeteners, flavoring agents, softeners, emulsifiers, colors, acidulants, buffering agents, fillers, antioxidants, and other components that provide desired attributes.

Combinations of sugar and/or non-sugar sweeteners can be used in the chewing gum formulation processed in accordance with the invention. Additionally, the softener may also provide additional sweetness such as aqueous sugar or alditol solutions.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.
High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another chewing gum component such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0 to about 8% by weight, preferably 0.001 to about 5% by weight.

If a low-calorie gum is desired, a low-caloric bulking agent can be used. Examples of low caloric bulking agents include polydextrose, Raftilose, Raftilin, fructooligosaccharides (NutraFlora^{®}), palatinose oligosaccharides; guar gum hydrolysates (e.g. Sun Fiber^{®}) or indigestible dextrins (e.g. Fibersol^{®}). However, other low-calorie bulking agents can be used.

The compressed chewing gum tablet according to the present invention may comprise aroma agents and flavoring agents prior to film coating, including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

The chewing gum flavor comprised in the tablet may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors may also be used in the present compressed chewing gum tablets. As indicated above, the aroma agent may be used in quantities smaller than those conventionally used. The aroma agents and/or flavors may be used in the amount from 0.01 to about 30% by weight of the final product depending on the desired intensity of the aroma and/or flavor used. Preferably, the content of aroma/flavor is in the range of 0.2 to 5%, more preferably 0.5 to 3%, by weight of the total composition.

In an example of the invention, the flavoring agents comprise natural and synthetic flavorings in the form of natural vegetable components, essential oils, essences, extracts, powders, including acids and other substances capable of affecting the taste profile.

Preferably, the liquid flavoring may be incorporated in the film coating is used to match the initial burst of sweetener release form the compressed chewing gum tablet. Also, the film coating itself may initially delay the burst of sweetness by keeping the chewing gum particles isolated from saliva thereby providing a more balanced release of sweetener from the tablet and liquid flavor from the film coating.

In a further example of the invention, the liquid flavoring comprised in the film coating may be used as taste masking in chewing gum comprising active ingredients, which by themselves have undesired taste or which alter the taste of the formulation. The film coating of the compressed chewing gum tablet may initially assist in the taste masking by delaying the chewer's perception of the undesired taste through fast release of flavor from the film coating.

Further chewing gum ingredients, which may be included in the compressed chewing gum tablet gum according to provisions of the present invention, include surfactants and/or solubilizers, especially when pharmaceutically or biologically active ingredients are present. As examples of types of surfactants to be used as solubilizers in a chewing gum composition according to examples of the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilizers can be used. Suitable solubilizers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilizers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubilizer may either be a single compound or a combination of several compounds.

Antioxidants may include materials that scavenge free radicals. In some examples, antioxidants include but are not limited to ascorbic acid, citric acid, rosemary oil, vitamin A, vitamin E, vitamin E phosphate, tocopherols, di-alpha- tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some examples, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some examples, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E161g), cryptoxanthin (E161c), rubixanthin (E161d), violanxanthin (E161e), rhodoxanthin (E161f), caramel (E150(a-d)), P-apo-8'-carotenal (E160e), β-carotene, (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some examples, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some examples, certified colors can include FD&C aluminum lakes. These consist of the aluminum salts of FD&C dyes extended on an insoluble substrate of alumina hydrate. Additionally, in some examples, certified colors can be included as calcium salts.

Active ingredients may advantageously be applied in a chewing gum according to the invention. Active ingredients generally refer to those ingredients that are included in a chewing gum composition for the desired end benefit they provide to the user. In some examples, active ingredients can include medicaments, nutrients, nutraceuticals, herbals, nutritional supplements, pharmaceuticals, drugs, and the like and combinations thereof. Moreover, in the present context, active ingredients may refer to flavor components, high intensity sweeteners or other taste establishing components.

Preferred active pharmaceutical ingredients (API) to be used in chewing gum according to examples of the invention are selected from the groups of antihistamines, anti-smoking agents, agents used for diabetes, decongestrants, peptides, pain-relieving agents, antacids, nausea-relieving agents, statines, and other.

Most preferred API according to examples of the invention are cetirizine, levo cetirizine, nicotine, nicotine polacrilex, nicotine in combination with alkaline agents, metformin, metformin HCL, phenylephrine, GLP-1, exenatide, MC-4 receptor antagonist, PYY(3-36), deca-peptide, KSL-W (acetate), fluor, and chlorhexidine.

Also preferred API according to examples of the invention are loratadine, desloratadine, nicotine bitartrate, nicotine in combination with caffeine, nicotine antagonists, combinations thereof or compounds comprising one or more of these, pseudoephedrine, flurbiprofen, paracetamol, acetylsalicylic acid, Ibuprofen, antacida, cimetidine, ranitidine, ondansetron, granisetron, metoclopramid, simvastatin, lovastatin, fluvastatin, acyclovir, benzydamin, rimonabant , varenicline, sildenafil, naltrexone, fluor in combination with fruit acids, derivatives, salts or isomers of chlorhexidine.

Some groups of suitable enhancers to e.g. enhance the uptake of API include bile salts, cetomacrogols, chelating agents, citrates, cyclodextrins, detergents, enamine derivatives, fatty acids, labrasol, lecithins, phospholipids, synthetic and natural surfactants, nonionic surfactants, cell envelope disordering compounds, solvents, steroidal detergents, chelators, solubilization agents, charge modifying agents, pH control agents, degradative enzyme inhibitors, mucolytic or mucus clearing agents.

Further groups of suitable enhancers include modulatory agents of epithelial junction physiology such as nitric oxide (NO) stimulators, chitosan, and chitosan derivatives; and vasodilator agents, selective transport-enhancing agents, stabilizing delivery vehicles, carriers, supports or complex- forming species with which exendins may be effectively combined, associated, contained, encapsulated or bound to stabilize an active agent for enhanced mucosal delivery; and membrane penetration-enhancing agents including surfactants, bile salts, phospholipid or fatty acid additives, mixed micelle, liposome, carrier, alcohol, enamine, NO donor compound, a long-chain amphipathic molecule, small hydrophobic penetration enhancer, sodium or a salicylic acid derivative, glycerol ester of acetoacetic acid, cyclodextrin or beta-cyclodextrin derivative, medium-chain fatty acid, chelating agent, amino acid or salt thereof, N-acetylamino acid or salt thereof, enzyme degradative to a selected membrane component, inhibitor of fatty acid synthesis, inhibitor of cholesterol synthesis, any combination of the membrane penetration enhancing agents.

Examples of enhancers suitable for application in chewing gum according to examples of the invention include cetylpyridinium chloride (CPC), benzalkonium chloride, sodium lauryl sulfate, polysorbate 80, Polysorbate 20, cetyltrimethylammonium bromide, laureth 9, sodium salicylate, sodium EDTA, EDTA, aprotinin, sodium taurocholate, saponins, bile salt derivatives, fatty acids, sucrose esters, azone emulsion, dextran sulphate, linoleic acid, labrafil, transcutol, urea, azone, nonionic surfactants, sulfoxides, sauric acid/PG, POE 23 lauryl ether, methoxysalicylate, dextran sulfate, methanol, ethanol, sodium cholate, Sodium taurocholate, Lysophosphatidyl choline, Alkylglycosides, polysorbates, Sorbitan esters, Poloxamer block copolymers, PEG-35 castor oil, PEG-40 hydrogenated castor oil, Caprocaproyl macrogol-8 glycerides, PEG-8 caprylic/capric, glycerides, Dioctyl sulfosuccinate, Polyethylene lauryl ether, Ethoxydiglycol, Propylene glycol, mono-di-caprylate, Glycerol monocaprylate, Glyceryl fatty acids (C.sub.8-C.sub.18) ethoxylated, Oleic acid, Linoleic acid, Glyceryl caprylate/caprate, Glyceryl monooleate, Glyceryl monolaurate, Capryliccapric triglycerides, Ethoxylated nonylphenols, PEG-(8-50) stearates, Olive oil PEG-6, esters, Triolein PEG-6 esters, Lecithin, d-alpha tocopherol polyethylene glycol 1,000 succinate, Citric acid, Sodium citrate, BRIJ, Sodium laurate, 5-methoxysalicylic acid, Bile salts, Acetyl salicylate, ZOT, Docosahexaenoic acid, Alkylglycosides, Sodium glycocholate (GC-Na), Sodium taurocholate (TC-Na), EDTA, Choline salicylate, Sodium caprate (Cap-Na), N-lauryl-beta-D-maltopyranoside (LM), Diethyl maleate, Labrasol, Sodium salicylate, Menthol, Alkali metal alkyl sulphate, Sodium lauryl sulphate, Glycerin, Bile acid, Lecithin, phosphatidylcholine, phosphatidylserine, sphingomyelin, phophatidylethanolamine, cephalin, lysolecithin, Hyaluronic acid: alkalimetal salts, sodium, alkaline earth and aluminum, Octylphenoxypolyethoxyethanol, Glycolic acid, Lactic acid, Chamomile extract, Cucumber extract, Borage oil, Evening primrose oil, Polyglycerin, Lysine, Polylysine, Triolein, Monoolein, Monooleates, Monolaurates, Polydocanol alkyl ethers, Chenodeoxycholate, Deoxycholate, Glycocholic acid, Taurocholic acid, Glycodeoxycholic acid, Taurodeoxycholic acid, Sodium glycocholate, Phosphatidylcholine, Phosphatidylserine, Sphingomyelin, Phosphatidylethanolamine, Cephalin, Lysolecithin, Alkali metal hyaluronates, Chitosan, Poly-L-arginine, Alkyl glucoside, Saccharide alkyl ester, Fusidic acid derivatives, Sodium taurdihydrofusidate (STDHF), L-α-phosphatidylcholine Didecanoyl (DDPC), Nitroglycerine, nitropruside, NOC5 [3-(2-hydroxy-1-(methyl-ethyl)-2-nitrosohydrazino)-1- propanamine], NOC12 [iV-ethyl-2-(1-ethyl-hydroxy-2-nitrosohydrazino)-ethanamine, SNAP [S-nitroso-N-acetyl-DL-penicillamine, NORI, NOR4, deacylmethyl sulfoxide, azone, salicylamide, glyceryl-1,3-diacetoacetate, 1,2-isopropylideneglycerine-3-acetoacetate), Amino acids, Amino acid salts, monoaminocarboxlic acids, Glycine, alanine, phenylalanine, proline, hydroxyproline, hydroxyamino acids, serine, acidic amino acids, aspartic acid, Glutamic acid, Basic amino acids, Lysine, N-acetylamino acids, N-acetylalanine, N-acetylphenylalanine, TM-acetylserine, N-acetylglycine, N-acetyllysine, N-acetylglutamic acid, N-acetylproline, N-acetylhydroxyproline , lactic acid, malic acid and citric acid and alkali metal salts thereof, pyrrolidonecarboxylic acids, alkylpyrrolidonecarboxylic acid esters, N-alkylpyrrolidones, proline acyl esters, sodium lauryl phosphate, sodium lauryl sulphate, sodium oleyl phosphate, sodium myristyl sulphate, polyoxyethylene alkyl ethers, polyoxyethylene alkyl esters, and caproic acid, alkylsaccharide, fusidic acid, polyethylene glycol, cetyl alcohol, polyvinylpyrolidone, Polyvinyl alcohol, Lanolin alcohol, Sorbitan monooleate, Ethylene glycol tetraacetic acid, Bile acid conjugate with taurine, Cholanic acid and salts, Cyclodextran, Cyclodextrin, Cyclodextrin (beta), Hydroxypropyl-β-cyclodetran, Sulfobutylether-β-cyclodextran, Methyl-β-cyclodextrin, Chitosan glutamate, Chitosan acetate, Chitosan hydrochloride, Chitosan hydrolactate, 1-O-alkyl-2-hydroxy-sn-glycero-3-phosphocholine, 3-O-alkyl-2-acetoyl-sn-glycero-1-phosphocho line, 1-O-alkyl-2-O-acetyl-sn-glycero-3-phospho(N,N,N-trimethyl)hexanolamine, propylene glycol, tetradecylmaltoside (TDM), sucrose dedecanoate.

Examples of suitable mucoadhesives as enhancers according to examples of the invention include Carbopol 934+HPC, Maize + Carbopol 907, HPC (hydroxypropyl cellulose), Na-CMC, HPMC (hydroxypropylmethylcellulose), HEMA hydroxyethyl metacrylate, Carbopol 907 crosslinked with sucrose, Polyacrylic acids (PAA), Chitosans, Lectins, Polymetacrylate derivatives, Hyaluronic acid, P(AA-co-PEG) monomethylether monomethacrylate, PAA-PVP (Poly acrylic acid-poly vinyl pyrrilidone), PVP-PEG, methylcellulose, N-Trimethyl Chitosans, PDMAEMA, poly(dimethyl-aminoethyl methacrylate), HEC Hydroxethyl Cellulose, Carbomer 940, Carbomer 971, Polyethylene Oxide, Dextrin, Poly(Methyl Vinyl Ether/Maleic Anhydride), Polycarbophil that is polymers of acrylic acid crosslinked with divinyl glycol, PVP (PVP: Poly vinyl pyrrilidone), Agar, Tragacanth, Sodium Alginate, Karaya gum, MEC, HPC (HPC: Hydroxy propyl cellulose), Lectins, AB Block copolymer of oligo (methyl methacrylate) and PAA, Polymers with thiol groups, Spheromers, Thiomers, Alginic acid sodium salt, Carbopol 974P (Carbomer), EC (EC: Etylcellulose), CMC (CMC: Carboxymethyl cellulose), Dextran, Guar Gum, Pectins, Starch, Gelatin, Casein, Acrylic acid polymers, Polymers of acrylic acid esters, Acrylic acid copolymers, Vinyl polymers, Vinyl copolymers, Polymers of Vinyl alcohols, Alcoxy polymers, polyethylene oxide polymers, and polyethers.

Some groups of suitable enhancers include solubilization agents; charge modifying agents; pH control agents; degradative enzyme inhibitors; modulatory agents of epithelial junction physiology, such as nitric oxide (NO) stimulators, chitosan, or chitosan derivatives; vasodilator agents; selective transport-enhancing agents; stabilizing delivery vehicles, carriers, supports or complex- forming species with which exendin(s) is/are effectively combined, associated, contained, encapsulated or bound to stabilize the active agent for enhanced mucosal delivery; small hydrophilic penetration enhancers; emulsifiers, mucolytic or mucus clearing agents; membrane penetration-enhancing agents such as e.g., (i) a surfactant, (ii) a bile salt, (iii) a phospholipid or fatty acid additive, mixed micelle, liposome, or carrier, (iv) an alcohol, (v) an enamine, (iv) an NO donor compound, (vii) a long-chain amphipathic molecule, (viii) a small hydrophobic penetration enhancer, (ix) sodium or a salicylic acid derivative, (x) a glycerol ester of acetoacetic acid, (xi) a cyclodextrin or beta-cyclodextrin derivative, (xii) a medium-chain fatty acid, (xiii) a chelating agent, (xiv) an amino acid or salt thereof, (xv) an N-acetylamino acid or salt thereof, (xvi) an enzyme degradative to a selected membrane component, (xvii) an inhibitor of fatty acid synthesis, (xviii) an inhibitor of cholesterol synthesis; or (xiv) any combination of the membrane penetration enhancing agents of (i)-(xviii)).
In various examples of the invention, exendin is combined with one, two, three, four or more of the mucosal delivery-enhancing agents recited above.

Some suitable enhancers for application according to the present invention include pH control agents selected from the group consisting of Acetic acid, Adipic acid, Citric acid, Fumaric acid, Glucono-δ-lactone, Gluconic acid, Lactic acid, Malic acid, Maleic acid, Tartaric acid, Succinic acid, Propionic acid, Ascorbic acid, Phosphoric acid, Sodium orthophosphate, Potassium orthophosphate, Calcium orthophosphate, Sodium diphosphate, Potassium diphosphate, Calcium diphosphate, Pentasodium triphosphate, Pentapotassium triphosphate, Sodium polyphosphate, Potassium polyphosphate, Carbonic acid, Sodium carbonate, Sodium bicarbonate, Potassium carbonate, Calcium carbonate, Magnesium carbonate, Magnesium oxide, or any combination thereof.

The suitable pH control agents suitable according to the present invention include buffers.

In the present context, the terms granule and particle are used interchangeable in the sense that a granule or particle for use in a compression process is regarded to be a relatively small object, which together with other granules or particles may be compressed into a stable chewing gum tablet. The granules or particles may be produced in several different ways. A gum base-containing granule or particle may typically be produced substantially into the desired shape by means of an extrusion process or alternatively be produced on the basis of a gum base-containing mass which is subsequently separated into particles of a smaller size.

The following non-limiting examples illustrate different variations of film coated compressed chewing gum tablets according to examples of the invention. The examples are meant for indicating the inventive concept; hence the mentioned examples should not be understood as exhaustive for the present invention.
The invention relates to a compressed chewing gum tablet comprising at least one chewing gum module, the chewing gum module including a chewing gum composition comprising chewing gum particles containing gum base, wherein the chewing gum tablet is provided with a film coating comprising liquid flavoring.

### EXAMPLES

### EXAMPLE 1

### Preparation of gum base

The applied gum base used had the following composition and was mixed in a conventional mixing process:

| | |
|---|---|
| elastomer: | 19% by weight |
| natural resin: | 20% by weight |
| synthetic resin: | 20% by weight |
| fat/fillers: | 26% by weight |
| wax: | 15% by weight |

Obviously within the scope of the invention gum base may be prepared by other processes such as a one-step process or any other conventional process.

### EXAMPLE 2

### Preparation of chewing gum granules

The gum base of example 1 was used in the manufacture of chewing gum products according to embodiments of the invention.

An extruder (Leistritz ZSE/BL 360 kw 104, available from Leistritz GmbH, Germany) extruded the composition through the die plate into the liquid filled chamber (granulator A5 PAC 6, available from GALA GmbH, Germany). Descriptions of the extruder and the granulator may be found in e.g. WO 2004/098305, incorporated herein by reference.

Gum base in the form of pellets and menthol flavor crystals (MENTHOL BPIUSP, available from SHARP MENTHOL INDIA LIMITED, India), were mixed in the extruder. The chewing gum composition had the composition as shown in table 1.

**Table 1.**

| **Ingredient** | **Amount (wt%)** |
|---|---|
| gum base | 97 |
| menthol flavor crystals | 3 |

The extruder delivered the composition at a feed rate of 400 kg/h to the die plate. The extruder screw speed was 247 rpm. The minimum temperature in the extruder was 44°C and a temperature of less than 70°C was maintained along about ¾ of the extruder barrel length, until the composition passed the heating device in the outlet end of the extruder. Here the composition was heated to an extruder exit temperature of 109°C. The extruder and the granulator produced a pressure difference of 71 bar.

The composition was extruded through the die plate, which was heated to a temperature of 177°C and had 696 holes with a diameter of 0.36mm. In the granulator chamber the extruded composition was cut to granules by a cutter with 8 blades and cutter speed 1999 rpm. The particles were cooled and transported to the strainer unit (a centrifugal dryer TWS 20, available from GALA GmbH, Germany) in water with temperature 11°C and flow 22 m³/h. The average cooling and transport time in water was approx. 60 seconds. The particle rate was 400 kg/h and the average diameter of the obtained particles was 0.93mm.

The cooling and transport stage carried out in water in this example could be carried out in other media as well such as e.g. air.

Finally, talc was attached to the granules in between de-watering and conveyance to the tablet pressing apparatus or storing or packaging e.g. for transportation.

It should be emphasized that the above-applied manufacturing methods of gum base-containing granules are only exemplary and although advantageous not mandatory. Thus, an alternative method for the manufacturing of gum base-containing granules may be a conventional process involving that an initial gum base-containing mass is cooled to a low temperature, preferably to a temperature below 0°C and then mechanically particulated into small relatively irregular gum base-containing granules. If desired, these particles may be sieved or further processed to obtain a homogeneous granule blend. An example of such alternative process is disclosed in WO 2004/073691 as applied in a multi-layer tablet, hereby incorporated by reference.

### EXAMPLE 3

### Preparation of a single layer compressed chewing gum tablet

The composition of particles from Example 2 was compressed into a tablet according to the following process:
High-intensity sweeteners: aspartame powder and acesulfame K; bulk sweetener: Xylitab; and further ingredients were mixed into a homogenous blend of particles in a standard mixer:

The resulting composition is seen in table 2.

**Table 2.**

| **Mixture for pressed tablets** | |
|---|---|
| **Ingredient** | **Wt%** |
| Chewing gum granules | 40.0 |
| High intensity sweeteners | 0.35 |
| Miscellaneous | 4.9 |
| Xylitab | 50.65% |
| Peppermint blend | 3.0 |
| Menthol | 1.1 |

Before pressing, the mixtures passed a standard horizontal vibration sieve removing particles larger than 2.6 mm. The mixture was lead to a standard tablet pressing machine comprising dosing apparatus (P 3200 C, available from Fette GmbH, Germany) and pressed into compressed tablets.

Optionally a pre-compression step may be used prior to the compression step.
The weight of the individual tablets was about 1.3 g.

### EXAMPLE 4

### Film coating of a compressed chewing gum tablet

Dry film composition by percentages (w/w):

| | |
|---|---|
| HPMC: | 68.5% |
| Peppermint liquid flavor: | 25.0% |
| PEG 400: | 6.0% |
| Emulsifier: | 0.5% |

The film coating was applied using a DRIA Coater 1200 and 2 Schlick spray guns. 30 kg of tablets from example 3 were charged into the coater.
The application rate was about 150 ml/min (average) and the atomizing air pressure about 3.0 bar. The inlet temperature was about 50°C and the relative humidity of the inlet air approximately 10 %.
The drying time was about 15 minutes.
The coating level was about 3% by weight of the tablet and the coating comprised about 85% of water as a solvent prior to application.

Upon taste evaluation of the film coated compressed chewing gum tablet it was found that the disintegration of the tablet in the initial chewing phase was less pronounced when compared to an uncoated tablet. Furthermore, when compared to an uncoated chewing gum tablet, the taste of the film coated compressed chewing gum tablet was moderated in such a way that the burst of sweetness form the tablet granules was balanced by the liquid peppermint flavor in the film coating. The overall taste sensation was thus attenuated towards greater acceptance and longer taste.

### EXAMPLE 5

### Film coating of a compressed chewing gum tablet

Dry film composition by percentages (w/w):

| | |
|---|---|
| Ethylcellulose: | 60.0% |
| Plastcizer: | 14.5% |
| Orange Flavor: | 25.0% |
| Emulsifier: | 0.5% |

The film coating was applied as described in example 4.
The coating level was about 3% by weight of the tablet.

Upon taste evaluation of the film coated compressed chewing gum tablet it was found that the disintegration of the tablet in the initial chewing phase was less pronounced when compared to an uncoated tablet. Furthermore, when compared to an uncoated chewing gum tablet, the taste of the film coated compressed chewing gum tablet was moderated in such a way that the burst of sweetness form the tablet granules was balanced by the liquid peppermint flavor in the film coating. The overall taste sensation was thus attenuated towards greater acceptance and longer taste.

### EXAMPLE 6

### Film coating of a compressed chewing gum tablet comprising nicotine

To obtain a compressed chewing gum tablet comprising nicotine, the composition of example 3 was altered in such a way that 1% of the bulk sweetener (Xylitab) was replaced with nicotine polacrilex.
After compression, the resulting tablets were coated with a coating of the following composition, percentages by weight:

| | |
|---|---|
| HPMC: | 69% |
| Orange flavor | 30% |
| Tween 80 | 1.0% |

The film coating was applied as described in example 4.
The coating level was about 2.5% by weight of the tablet.

Upon taste evaluation of the film coated compressed chewing gum tablet it was found that the disintegration of the tablet in the initial chewing phase was less pronounced when compared to an uncoated tablet. Furthermore, when compared to an uncoated chewing gum tablet, the bitter taste of nicotine was masked efficiently by the combined effects of the flavor in the film coating and the sweetness burst from the sweeteners comprised in the tablet granules.

### EXAMPLE 7

### Film coating of a compressed chewing gum tablet comprising nicotine.

To obtain a compressed chewing gum tablet comprising nicotine, the composition of example 3 was altered in such a way that 1% of the bulk sweetener (Xylitab) was replaced with nicotine polacrilex.
After compression, the resulting tablets were coated with a coating of the following composition, percentages by weight:

| | |
|---|---|
| Cellulose acetate phthalate: | 59.0% |
| Diethyl phthalate: | 13.5% |
| Orange Flavor: | 25.0% |
| Emulsifier: | 0.5% |

The film coating was applied as described in example 4.
The coating level was about 3.0 % by weight of the tablet.

Upon taste evaluation of the film coated compressed chewing gum tablet it was found that the disintegration of the tablet in the initial chewing phase was less pronounced when compared to an uncoated tablet. Furthermore, when compared to an uncoated chewing gum tablet, the bitter taste of nicotine was masked efficiently by the combined effects of the flavor in the film coating and the sweetness burst from the sweeteners comprised in the tablet granules.

### EXAMPLE 8

### Film coating of a compressed chewing gum tablet

Dry film composition by percentages (w/w):

| | |
|---|---|
| HPMC: | 89.5% |
| PEG 400: | 7.8% |
| Peppermint Flavor: | 2.5% |
| Emulsifier: | 0.2% |

The film coating was applied as described in example 4.
The coating level was about 3% by weight of the tablet.

Upon taste evaluation of the film coated compressed chewing gum tablet it was found that the disintegration of the tablet in the initial chewing phase was less pronounced when compared to an uncoated tablet.
Even with this comparatively low flavor content in the film coating, an improved match of the sweetness burst from the tablet granules was seen when compared to an uncoated tablet.

### EXAMPLE 9

### Film coating of a compressed chewing gum tablet

Dry film composition by percentages (w/w):

| | |
|---|---|
| Ethylcellulose: | 72.5% |
| Plasticizer: | 24.8% |
| Orange Flavor: | 2.5% |
| Emulsifier: | 0.2% |

The film coating was applied as described in example 4.
The coating level was about 3% by weight of the tablet.

Upon taste evaluation of the film coated compressed chewing gum tablet it was found that the disintegration of the tablet in the initial chewing phase was less pronounced when compared to an uncoated tablet.
Even with this comparatively low flavor content in the film coating, an improved match of the sweetness burst from the tablet granules was seen when compared to an uncoated tablet.

## Claims

1. Compressed chewing gum tablet comprising at least one compressed chewing gum module, the at least one compressed chewing gum module including a compressed particulate chewing gum composition, which compressed particulate chewing gum composition comprises compressed chewing gum particles containing gum base,
wherein the content of gum base is at least 5 % by weight of the tablet, and wherein the chewing gum tablet is provided with a film coating,
wherein the film coating comprises liquid flavoring in an amount of more than 1% by weight of the dry film coating and
wherein the amount of liquid flavor in the chewing gum tablet, including the film coating, is above 0.05 % by weight of the chewing gum tablet.

2. Compressed chewing gum tablet according to claim 1, wherein the amount of liquid flavor in the chewing gum tablet, excluding the film coating, is below 2 %, such as below 1%, by weight of the chewing gum tablet.

3. Compressed chewing gum tablet according to claim 1 or 2, wherein any liquid flavor in the chewing gum tablet, excluding the film coating, is located in the compressed chewing gum particles containing gum base.

4. Compressed chewing gum tablet according to any of the claims 1-3, wherein said compressed chewing gum tablet comprises at least one active pharmaceutical ingredient.

5. Compressed chewing gum tablet according to any of the claims 1-4, wherein said film coating is applied to said compressed chewing gum tablet prior to any optional coating.

6. Compressed chewing gum tablet according to any of the claims 1-5, wherein the liquid flavor in the film coating constitutes at least 10% (w/w), preferably at least 20% (w/w) of the total flavor content of the film coated chewing gum tablet.

7. Compressed chewing gum tablet according to any of the claims 1-6, wherein said liquid flavoring is selected from the group consisting of coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, mint, fruit essence such as from apple, pear, peach, strawberry, apricot, raspberry, cherry, pineapple, and plum essence, essential oils including peppermint, spearmint, menthol, eucalyptus, clove oil, bay oil, anise, thyme, cedar leaf oil, nutmeg, oils of the fruits mentioned above and combinations thereof.

8. Compressed chewing gum tablet according to any of the claims 1-7, wherein said film coating comprises liquid flavoring in an amount of more than 5% by weight of the dry film coating.

9. Compressed chewing gum tablet according to any of the claims 1-8, wherein said film coating confers a modified release performance with respect to active components of the chewing gum formulation.

10. Compressed chewing gum tablet according to any of the claims 1-9, wherein said film coating comprises high intensity sweetener.

11. Compressed chewing gum tablet according to any of the claims 4-10, wherein said at least one active pharmaceutical ingredient is nicotine.

12. Compressed chewing gum tablet according to any of the claims 4-10, wherein said at least one active pharmaceutical ingredient is selected from the group consisting of cetirizine, levo cetirizine, metformin, metformin HCL, phenylephrine, GLP-1, exenatide, MC-4 receptor antagonist, PPY(3-36), deca-peptide, KSL-W (acetate), fluor, and chlorhexidine.

13. Compressed chewing gum tablet according to any of the claims 1-12, wherein said film coating comprises at least one active pharmaceutical ingredient.

14. Compressed chewing gum tablet according to any of the claims 1-13, wherein said compressed chewing gum tablet comprises biodegradable polymers.

15. Method for production of a chewing gum according to any of the claims 1-14, wherein said method comprises at least one compression step and at least one film coating step.

## Patentansprüche

1. Verdichtete Kaugummi-Tablette, welche mindestens ein verdichtetes Kaugummimodul umfasst, wobei das mindestens eine verdichtete Kaugummimodul eine verdichtete teilchenförmige Kaugummizusammensetzung umfasst, wobei die verdichtete teilchenförmige Kaugummizusammensetzung verdichtete, Gummigrundlage enthaltende Kaugummiteilchen umfasst, wobei der Gehalt an Gummigrundlage mindestens 5 Gew.-% der Tablette beträgt und wobei die Kaugummi-Tablette mit einem Filmüberzug versehen ist,
wobei der Filmüberzug flüssigen Geschmack- oder Aromastoff in einer Menge von mehr als 1 Gew.-% des trockenen Filmüberzugs umfasst und
wobei die Menge von flüssigem Geschmack- oder Aromastoff in der Kaugummi-Tablette, einschließlich des Filmüberzugs, über 0,05 Gew.-% der Kaugummi-Tablette liegt.

2. Verdichtete Kaugummi-Tablette nach Anspruch 1, wobei die Menge von flüssigem Geschmack- oder Aromastoff in der Kaugummi-Tablette, ausschließlich des Filmüberzugs, unter 2 Gew.-%, wie unter 1 Gew.-% der Kaugummi-Tablette liegt.

3. Verdichtete Kaugummi-Tablette nach Anspruch 1 oder 2, wobei ein jeglicher flüssiger Geschmack- oder Aromastoff in der Kaugummi-Tablette, ausschließlich des Filmüberzugs, sich in den verdichteten, Gummigrundlage enthaltenden Kaugummiteilchen befindet.

4. Verdichtete Kaugummi-Tablette nach einem der Ansprüche 1-3, wobei die verdichtete Kaugummi-Tablette mindestens einen aktiven pharmazeutischen Inhaltsstoff umfasst.

5. Verdichtete Kaugummi-Tablette nach einem der Ansprüche 1-4, wobei der Filmüberzug auf der verdichteten Kaugummi-Tablette vor einem jeglichen optionalen Überzug aufgebracht ist.

6. Verdichtete Kaugummi-Tablette nach einem der Ansprüche 1-5, wobei der flüssige Geschmack- oder Aromastoff in dem Filmüberzug mindestens 10% (Gew./Gew.), vorzugsweise mindestens 20% (Gew./Gew.) des gesamten Geschmack- oder Aromastoffgehalts der mit dem Filmüberzug überzogenen Kaugummi-Tablette ausmacht.

7. Verdichtete Kaugummi-Tablette nach einem der Ansprüche 1-6, wobei der flüssige Geschmack- oder Aromastoff ausgewählt ist aus der Gruppe bestehend aus Kokosnuss, Kaffee, Schokolade, Vanille, Grapefruit, Orange, Limette, Menthol, Süßholz, Karamelaroma, Honigaroma, Erdnuss, Walnuss, Cashew, Haselnuss, Mandel, Ananas, Erdbeere, Himbeere, tropischen Früchten, Kirschen, Zimt, Pfefferminze, Wintergrün, Krauseminze, Eukalyptus, Minze, Fruchtessenz wie aus Äpfeln, Birnen, Pfirsichen, Erdbeeren, Aprikosen, Himbeeren, Kirschen, Ananas und Pflaumenessenz, etherischen Ölen, einschließlich Pfefferminzöl, Krauseminzöl, Menthol, Eukalyptus, Nelkenöl, Lorbeeröl, Anis, Thymian, Zedernöl, Muskatnuss, Ölen der oben erwähnten Früchte und Kombinationen davon.

8. Verdichtete Kaugummi-Tablette nach einem der Ansprüche 1-7, wobei der Filmüberzug flüssigen Geschmack- oder Aromastoff in einer Menge von mehr als 5 Gew.-% des trockenen Filmüberzugs umfasst.

9. Verdichtete Kaugummi-Tablette nach einem der Ansprüche 1-8, wobei der Filmüberzug modifizierte Freisetzungseigenschaften in Bezug auf aktive Bestandteile der Kaugummiformulierung verleiht.

10. Verdichtete Kaugummi-Tablette nach einem der Ansprüche 1-9, wobei der Filmüberzug hochintensives Süßungsmittel umfasst.

11. Verdichtete Kaugummi-Tablette nach einem der Ansprüche 4-10, wobei der mindestens eine aktive pharmazeutische Inhaltsstoff Nicotin ist.

12. Verdichtete Kaugummi-Tablette nach einem der Ansprüche 4-10, wobei der mindestens eine aktive pharmazeutische Inhaltsstoff ausgewählt ist aus der Gruppe bestehend aus Cetirizin, Levocetirizin, Metformin, Metformin-HCl, Phenylephrin, GLP-1, Exenatid, MC-4-Rezeptor-Antagonist, PPY(3-36), Deca-peptid, KSL-W (Acetat), Fluor- und Chlorhexidin.

13. Verdichtete Kaugummi-Tablette nach einem der Ansprüche 1-12, wobei der Filmüberzug mindestens einen aktiven pharmazeutischen Inhaltsstoff umfasst.

14. Verdichtete Kaugummi-Tablette nach einem der Ansprüche 1-13, wobei die verdichtete Kaugummi-Tablette biologisch abbaubare Polymere umfasst.

15. Verfahren zur Herstellung eines Kaugummis nach einem der Ansprüche 1-14, wobei das Verfahren mindestens einen Verdichtungsschritt und mindestens einen Schritt eines Aufbringens eines Filmüberzugs umfasst.

## Revendications

1. Tablette de chewing-gum compressé, comprenant au moins un module de chewing-gum compressé, l'au moins un module de chewing-gum compressé comprenant une composition de chewing-gum particulaire compressé, ladite composition de chewing-gum particulaire compressé comprenant une base de gomme contenant des particules de chewing-gum compressé, dans laquelle la teneur en base de gomme est d'au moins 5 % en poids de la tablette, et dans laquelle la tablette de chewing-gum est fournie avec un pelliculage,
dans laquelle le pelliculage comprend un agent aromatisant liquide en une quantité supérieure à 1 % en poids du pelliculage sec et
dans laquelle la quantité d'arôme liquide dans la tablette de chewing-gum, comprenant le pelliculage, est supérieure à 0,05 % en poids de la tablette de chewing-gum.

2. Tablette de chewing-gum compressé selon la revendication 1, dans laquelle la quantité d'arôme liquide dans la tablette de chewing-gum, à l'exclusion du pelliculage, est inférieure à 2 % en poids, par exemple inférieure à 1 % en poids de la tablette de chewing-gum.

3. Tablette de chewing-gum compressé selon la revendication 1 ou 2, dans laquelle un arôme liquide quelconque dans la tablette de chewing-gum, à l'exclusion du pelliculage, se trouve dans la base de gomme contenant des particules de chewing-gum compressé.

4. Tablette de chewing-gum compressé selon l'une quelconque des revendications 1 à 3, dans laquelle ladite tablette de chewing-gum compressé comprend au moins un ingrédient pharmaceutique actif.

5. Tablette de chewing-gum compressé selon l'une quelconque des revendications 1 à 4, dans laquelle ledit pelliculage est appliqué sur ladite tablette de chewing-gum compressé avant tout pelliculage optionnel.

6. Tablette de chewing-gum compressé selon l'une quelconque des revendications 1 à 5, dans laquelle l'arôme liquide dans le pelliculage constitue au moins 10 % (p/p), de préférence, au moins 20 % (p/p) de la teneur en arôme totale de la tablette de chewing-gum pelliculée.

7. Tablette de chewing-gum compressé selon l'une quelconque des revendications 1 à 6, dans laquelle ledit arôme liquide est choisi dans le groupe comprenant les arômes de noix de coco, café, chocolat, vanille, pamplemousse, orange, citron vert, menthol, réglisse, caramel, miel, cacahuète, noix, noix de cajou, noisette, amande, ananas, fraise, framboise, fruits tropicaux, cerise, cannelle, menthe poivrée wintergreen, menthe verte, eucalyptus, menthe, des essences de fruit telles que des essences de pomme, poire, pêche, fraise, abricot, framboise, cerise, ananas et prune, des huiles essentielles comprenant les huiles de menthe poivrée, menthe verte, menthol, eucalyptus, clou de girofle, bay, anis, thym, cèdre, muscade, les huiles des fruits mentionnés ci-dessus et leurs combinaisons.

8. Tablette de chewing-gum compressé selon l'une quelconque des revendications 1 à 7, dans laquelle ledit pelliculage comprend un agent aromatisant liquide en une quantité supérieure à 5 % en poids du pelliculage sec.

9. Tablette de chewing-gum compressé selon l'une quelconque des revendications 1 à 8, dans laquelle ledit pelliculage confère une performance de libération modifiée par rapport aux composants actifs de la formulation de chewing-gum.

10. Tablette de chewing-gum compressé selon l'une quelconque des revendications 1 à 9, dans laquelle ledit pelliculage comprend un édulcorant de haute intensité.

11. Tablette de chewing-gum compressé selon l'une quelconque des revendications 4 à 10, dans laquelle ledit au moins un ingrédient pharmaceutique actif est la nicotine.

12. Tablette de chewing-gum compressé selon l'une quelconque des revendications 4 à 10, dans laquelle ledit au moins un ingrédient pharmaceutique actif est choisi dans le groupe comprenant la cétirizine, la lévo-cétirizine, la metformine, le chlorhydrate de metformine, la phényléphrine, GLP-1, l'exénatide, un antagoniste du récepteur MC-4, PPY(3-36), un décapeptide, KSL-W (acétate), le fluor et la chlorhexidine.

13. Tablette de chewing-gum compressé selon l'une quelconque des revendications 1 à 12, dans laquelle ledit pelliculage comprend au moins un ingrédient pharmaceutique actif.

14. Tablette de chewing-gum compressé selon l'une quelconque des revendications 1 à 13, dans laquelle ladite tablette de chewing-gum compressé comprend des polymères biodégradables.

15. Procédé de production d'un chewing-gum selon l'une quelconque des revendications 1 à 14, dans lequel ledit procédé comprend au moins une étape de compression et au moins une étape de pelliculage.
